# EUROPEAN PATENT APPLICATION

(11) **EP 4 763 246 A1**
(43) Date of publication of application: **24.06.2026**
(21) Application number: 25226928.7
(22) Date of filing: 23.12.2025
(51) Int. Cl.: A61M 5/14, A61B 90/57, A61M 25/02

(54) **SYSTEM FOR MANAGING PATIENT CARE MEDICAL LINES AND RELATED METHODS**

(30) Priority: 23.12.2024 US 202463737925 P
(71) Applicant: Laborie Medical Technologies Corp., Portsmouth, NH 03801 (US)
(72) Inventor: CHENGRIAN, Elizabeth Rose, Salt Lake City (US); BORJA, Marisa Janelle, San Francisco (US); CHEHAB, Eric Fayez, Oakland (US); FUERCH, Janene Hilary, Los Altos (US); BRODSKY, Sophia Esther, San Jose (US); CARTMILL, Tomas Jordan, Midvale (US); MUNGER, Jake Brenner, Herriman (US); NIPKO, Landon Kent, Salt Lake City (US)
(74) Representative: Reddie & Grose LLP

(57) **Abstract**

A medical line management system is provided that includes one or more line management modules configured to secure medical lines, such as fluid lines and respiratory lines, and a set of attachments configured to receive those modules at different locations. The line management module can be removably attached to a bedside attachment when the patient is in a bed or incubator and can also be removably attached to a family holding attachment when the patient is moved away from the bedside, such as for skin-to-skin or kangaroo care. In this way, the same line management module can travel with the patient while keeping the lines supported and organized at both the bedside and during holding.

## Description

### TECHNICAL FIELD

This disclosure relates generally to medical line management. More specifically, this disclosure relates to systems for organizing and managing medical lines to facilitate movement of a patient as well as supporting and organizing the lines in the bed and during holding.

### BACKGROUND

About 500,000 babies per year are admitted to neonatal intensive care units (NICUs) in the United States, for reasons ranging from extreme prematurity to congenital malformations of the heart and brain. These newborns are truly the hospital's most vulnerable patients, as they are on various forms of life support such as intravenous medication, nutrients, and respiratory support.

One strategy for treating patients that is unique to NICUs is family bonding. Studies have shown that physical interaction between neonates and their parents can improve neurodevelopmental outcomes and lead to shorter lengths of stay in the NICU. This is more commonly known as "skin-to-skin" or "kangaroo" care. However, nurses and physicians often hesitate to allow families to hold their baby out of fear of dislodging the various lines that are sustaining the baby's life. If families are allowed to hold their babies, nurses have adopted various tape-based and positional strategies to support the lines while the patient is being held. These are often unreliable, risky, and unstandardized between nurses and hospitals, and these may make it difficult to keep the lines properly organized.

### DISCLOSURE

In some embodiments, a medical line management system is provided that includes one or more line management modules configured to secure medical lines, such as fluid lines and respiratory lines. The line management module can be removably attached to a family holding attachment when the patient is moved away from a bedside, such as for skin-to-skin or kangaroo care. In this way, the same line management module can travel with the patient while keeping the lines supported and organized at both the bedside and during holding.

In another aspect, methods are described for managing and moving medical lines using a line management module and complementary attachments. Medical lines are first secured to the line management module, for example by using straps and securement knobs on the module to hold the lines in defined channels. When the patient is ready to be moved (e.g., for holding), the line management module is moved with the patient and attached to a family holding attachment, all while the medical lines remain secured to the line management module. The medical lines are thus maintained in an organized and supported arrangement throughout the transfer.

In a further aspect, a medical line management module is provided that can be mounted to external objects while holding medical lines in an organized configuration. The module includes a base with a plurality of line channels, each line channel configured to receive a medical line, and at least one line securement strap to retain each line in its channel. An attachment clip is connected to the base and is configured to removably mount the line management module to external supports, such as bedside attachments, family holding attachments, or belts and straps.

### BRIEF DESCRIPTION OF THE DRAWINGS

For a detailed understanding of the disclosure, reference should be made to the following detailed description, taken in conjunction with the accompanying drawings, in which like elements have generally been designated with like numerals. To easily identify the discussion of any particular element or act, the most significant digit or digits in a reference number refer to the figure number in which that element is first introduced.
FIG. 1 is a perspective view of an attachment clip for a medical line management system according to embodiments of the disclosure.
FIG. 2A is a perspective view of a fluid line management module of a medical line management system according to embodiments of the disclosure. FIG. 2B shows a side view of the fluid line management module of FIG. 2A, FIG. 2C shows a top view of the fluid line management module of FIG. 2A, and FIG. 2D shows an enlarged view of a securement knob of the fluid line management module of FIG. 2A.
FIG. 3A is a top perspective view of a respiratory line management module of a medical line management system, according to embodiments of the present disclosure, and FIG. 3B is a bottom perspective view of the respiratory line management module of FIG. 3A.
FIG. 4A is a perspective view of a bedside attachment of a medical line management system, and FIG. 4B shows the bedside attachment of FIG. 4A in an exemplary environment of use in accordance with embodiments of the present disclosure.
FIG. 5A is a side perspective view of a family holding attachment of a medical line management system, FIG. 5B is a top perspective view of the family holding attachment of FIG. 5A, and FIG. 5C is a top perspective view of the family holding attachment with an alternative clip securement column, according to embodiments of the present disclosure.
FIG. 6A is a perspective view of a sliding securement tab of a medical line management system, according to embodiments of the present disclosure, and FIG. 6B shows a side view of the sliding securement tab of FIG. 6A. FIG. 6C is a perspective view of a family holding attachment of a medical line management system.
FIG. 7 is a perspective view of a buckle for a family holding attachment of a medical line management system according to embodiments of the present disclosure.
FIG. 8A is a perspective view of a fluid line management module of a medical line management system according to embodiments of the present disclosure.
FIG. 8B is a perspective view of a fluid line management module of a medical line management system according to embodiments of the present disclosure.
FIG. 8C is a perspective view of a fluid line management module of a medical line management system according to embodiments of the present disclosure.
FIG. 8D is a perspective view of a fluid line management module of a medical line management system according to embodiments of the present disclosure.
FIG. 9 is a perspective view of a respiratory line management module of a medical line management system according to embodiments of the present disclosure.
FIG. 10 is a perspective view of a family holding attachment of a medical line management system.
FIG. 11 is a top perspective view of a respiratory line management module of a medical line management system, according to embodiments of the present disclosure.

### MODE(S) FOR CARRYING OUT THE INVENTION

The illustrations presented herein are not actual views of any medical line management system, line management module, bedside attachment, and family holding attachment for a line management module, or any component thereof, but are merely idealized representations, which are employed to describe embodiments of the invention.

As used herein, the singular forms following "a," "an," and "the" are intended to include the plural forms as well, unless the context clearly indicates otherwise.

As used herein, the term "may" with respect to a material, structure, feature, or method act indicates that such is contemplated for use in implementation of an embodiment of the disclosure, and such term is used in preference to the more restrictive term "is" so as to avoid any implication that other compatible materials, structures, features, and methods usable in combination therewith should or must be excluded.

As used herein, any relational term, such as "first," "second," "top," "bottom," "upper," "lower," "above," "beneath," "side," "upward," "downward," etc., is used for clarity and convenience in understanding the disclosure and accompanying drawings, and does not connote or depend on any specific preference or order, except where the context clearly indicates otherwise. For example, these terms may refer to an orientation of elements of any medical line management system, line management module, bedside attachment for a line management module, or component thereof when utilized in a conventional manner. Furthermore, these terms may refer to an orientation of elements of any medical line management system, line management module, bedside attachment, or family holding attachment for a line management module, or component thereof as illustrated in the drawings.

As used herein, the term "substantially" in reference to a given parameter, property, or condition means and includes to a degree that one skilled in the art would understand that the given parameter, property, or condition is met with a small degree of variance, such as within acceptable manufacturing tolerances. By way of example, depending on the particular parameter, property, or condition that is substantially met, the parameter, property, or condition may be at least 90.0% met, at least 95.0% met, at least 99.0% met, or even at least 99.9% met.

As used herein, the term "about" used in reference to a given parameter is inclusive of the stated value and has the meaning dictated by the context (e.g., it includes the degree of error associated with measurement of the given parameter, as well as variations resulting from manufacturing tolerances, etc.).

A medical line management system may be provided that easily manages and secures medical lines, such as fluid medical lines or respiratory medical lines, while also allowing movement of the medical lines from one location to another. This may facilitate, for example, family bonding (skin-to-skin or kangaroo care) in a neonatal care facility where medical lines may be safely managed at and moved between the neonate's bedside and near a parent or other care provider during family holding.

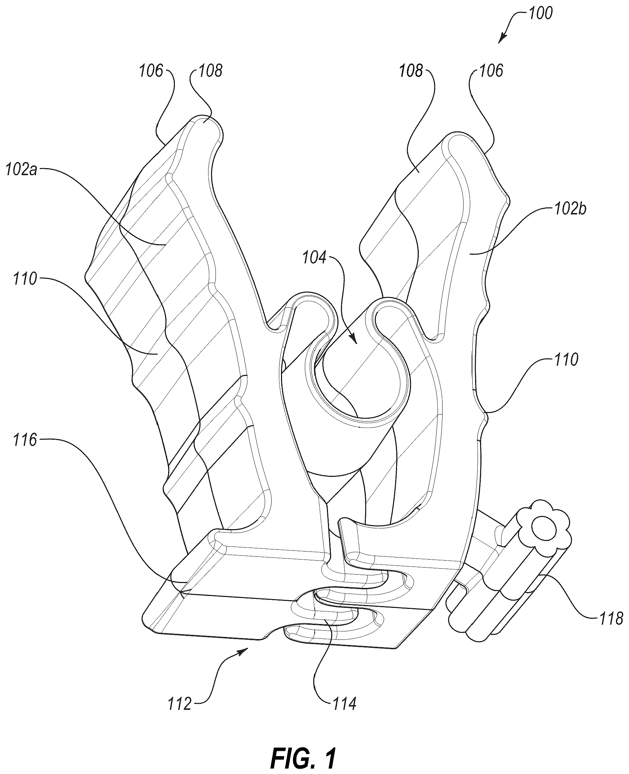

Referring to FIG. 1, an attachment clip 100 is illustrated. The attachment clip 100 is configured as a generally hand-operable clamp that can be coupled to various mounting structures and line-management modules of a medical line management system. The attachment clip 100 provides a standardized interface so that different modules can be secured to different supports while using a common clip geometry.

The attachment clip 100 includes a first clamping arm 102a and a second clamping arm 102b. The first clamping arm 102a and the second clamping arm 102b extend generally away from a central region of the clip and are arranged to move toward and away from one another to define an adjustable clamping opening. In the illustrated embodiment, the first clamping arm 102a and the second clamping arm 102b are formed as portions of a unitary body and are joined at a proximal region by a live joint 104. The live joint 104 is formed as a flexible, reduced-thickness hinge region that elastically bends when the clamping arms 102a, 102b are displaced relative to one another. The live joint 104 may have an "omega" shape as shown, though other shapes are also possible. The material properties and geometry of the live joint 104 bias the clamping arms 102a, 102b toward a closed position, so that the attachment clip 100 can resiliently grip a corresponding mounting structure when the arms are released.

Each clamping arm 102a, 102b is configured such that a distal end 106 forms a stopper 108. The stoppers 108 may be configured to project towards an interior of the attachment clip 100. When the clamping arms 102a, 102b are actuated to open the attachment clip 100, the distal ends 106 move toward one another until the stoppers 108 engage, thereby defining a maximum opening angle of the attachment clip 100. The position and geometry of the stoppers 108 are selected such that, at the maximum opening angle, strain in the live joint 104 remains within an elastic range of the clip material. Limiting the opening angle in this manner helps prevent plastic deformation or fatigue of the live joint 104 over repeated use while still providing a sufficiently large opening for engagement with the associated mounting structures.

Outer surfaces of the clamping arms 102a, 102b may be formed with grips 110 to facilitate manual operation of the attachment clip 100. The grips 110 can include a series of recessed regions, ribs, or other surface contours that receive the thumb and fingers of a user. The grips 110 can be sized and spaced based on expected hand sizes and on manufacturing constraints of a molding process and may include radiused transitions that improve comfort and reduce stress concentrations. In some embodiments, the grips 110 may incorporate texture, knurling, or locally increased friction to reduce the likelihood of slippage during use, allowing a caregiver to open the attachment clip 100 with one hand while positioning the associated module or mounting structure with the other hand.

The clamping arms 102a, 102b extend toward a lower base 112 of the attachment clip 100. The base 112 forms a region of the attachment clip 100 configured to interface with rails, grooves, or other complementary attachment features provided on different mounting structures of components of the medical line management system. The underside of the base 112 carries a plurality of interlocking teeth 114. The interlocking teeth 114 are arranged, for example, as a series of laterally spaced ridges or serrations that are configured to mate with corresponding teeth, grooves, or other engagement features on the cooperating mounting structure. The interlocking teeth 114 may also be configured to securely grip onto other materials such as fabric materials. When the attachment clip 100 is pressed onto such a structure, the interlocking teeth 114 engage the complementary features to inhibit sliding or rotation of the clip relative to the structure, thereby providing a positive mechanical interlock in addition to frictional engagement.

The base 112 also defines a foot 116 that extends from the base 112 and defines a lower bearing surface for the attachment clip 100. The foot 116 can provide a generally planar or slightly contoured contact region that allows the attachment clip 100 to stand on its own. In some embodiments, the foot 116 may be formed with a high-friction surface, a compliant pad, or a surface texture that increases resistance to unintended movement.

The attachment clip 100 further includes a connector 118 projecting from a side of the base 112 or one of the clamping arms 102a, 102b. The connector 118 is configured to mechanically couple the attachment clip 100 to a module or accessory of the medical line management system. For example, the connector 118 can be formed as a cylindrical boss, a multi-lobed shaft, or another keyed feature that is received in a complementary opening, track, or slot of the module or accessory. The geometry of the connector 118 can allow the associated module to pivot, translate, or otherwise reposition relative to the attachment clip 100 while the clip remains secured to a support structure by the base 112 and interlocking teeth 114. In some implementations, the connector 118 can define a rotational axis about which the module can be oriented between different use positions without detaching the attachment clip 100 from the underlying support.

In one implementation, the attachment clip 100 is formed as a single, integrally molded component from a resilient polymeric material, such as a medical-grade thermoplastic. The live joint 104 is defined by a locally reduced cross-section in the molded body, so that repeated flexing of the clamping arms 102a, 102b is accommodated primarily by elastic bending of this region. The stoppers 108, grips 110, base 112, interlocking teeth 114, foot 116, and connector 118 can all be formed in the same molding operation. In other embodiments, additional components, such as springs, pads, or inserts, may be attached to any of these features to further tailor the clamping force, frictional properties, or durability of the attachment clip 100.

In operation, a clinician can grasp the grips 110 on the first clamping arm 102a and the second clamping arm 102b and apply a squeezing or spreading force to elastically deform the live joint 104 and open the clamping arms 102a, 102b to the desired extent, up to the limit set by the stoppers 108. While the attachment clip 100 is held open, the base 112 is positioned against the corresponding rail, tab, or other mounting structure so that the interlocking teeth 114 are aligned with complementary features. When the clinician releases the grips 110, the live joint 104 urges the clamping arms 102a, 102b back toward the closed position, thereby clamping the attachment clip 100 onto the structure and engaging the interlocking teeth 114. A line-management module or other accessory can then be coupled to or removed from the connector 118 without disturbing the engagement between the attachment clip 100 and the underlying support.

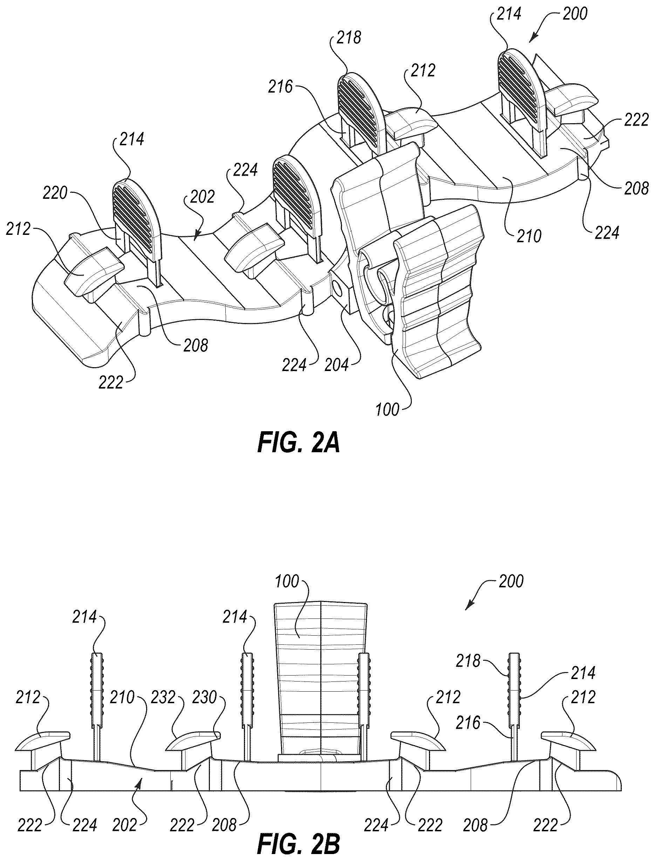

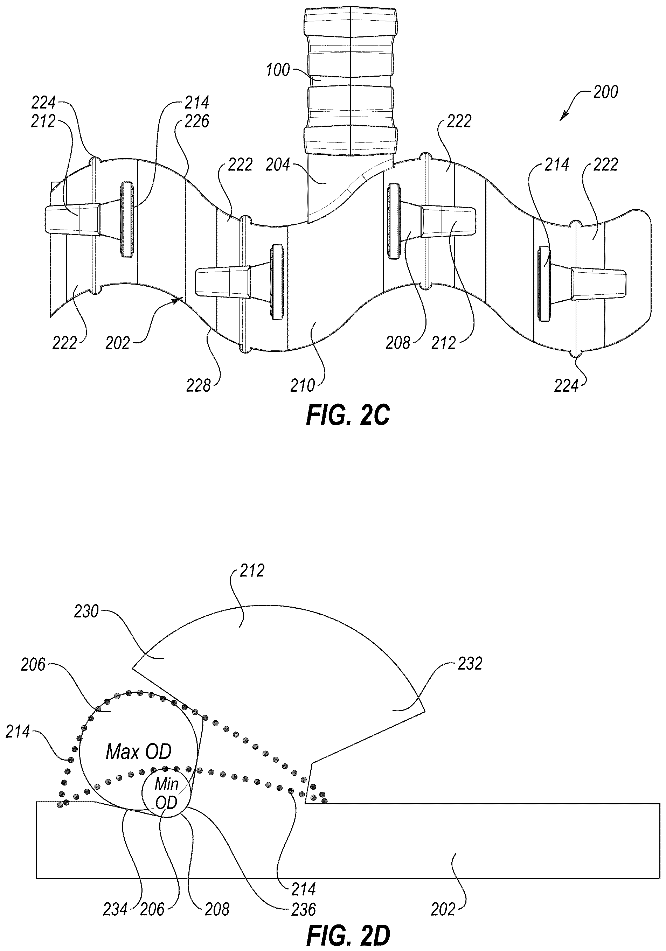

Referring to FIGS. 2A-2D, a fluid line management module 200 is illustrated. The fluid line management module 200 may be a part of a medical line management system. The fluid line management module 200 is configured to organize, hold, and manage a plurality of medical fluid lines 206. In the illustrated embodiment, the fluid line management module 200 includes a base 202 and an attachment clip 100 mounted to the base 202. The attachment clip 100 may be the attachment clip described above with reference to FIG. 1 and may be mounted to the base 202 at an attachment clip protrusion 204. The attachment clip 100 may be rotatably mounted to the attachment clip protrusion 204 so that the fluid line management module 200 can be attached to different support structures and used at different orientations while maintaining a convenient orientation of the fluid lines 206.

The base 202 defines a plurality of line channels 208 that each accommodate a respective fluid line 206 or cable. In the illustrated embodiment, the line channels 208 are arranged along a top surface 210 of the base 202. The base 202 and the top surface 210 have an undulating or "wavy" profile such that adjacent line channels 208 are laterally offset from one another. This wavy geometry increases the overall width and stability of the base 202 on a supporting surface and creates natural finger-placement regions between the line channels 208 to facilitate handling and visual access to individual fluid lines 206. The base 202 may be formed from a polymer-based material, such as a medical-grade plastic, by injection molding, additive manufacturing, or another suitable process.

Each line channel 208 is defined between a corresponding securement knob 212 and line securement strap 214. The securement knobs 212 and the line securement straps 214 protrude from the top surface 210 of the base 202. Each line securement strap 214 includes a securement portion 216 that extends upward from the base 202. The securement portion 216 may include two spaced, generally parallel members that are joined at a distal end by a finger grip 218. The finger grip 218 may have a friction-enhancing surface-such as ribs, ridges, or other texturing-to facilitate grasping and pulling of the line securement strap 214 by a clinician, including where a clinician may be wearing gloves. The securement portion 216 and finger grip 218 together define a slot 220 sized to receive the corresponding securement knob 212. In some embodiments, the securement knobs 212 and the line securement straps 214 are formed from an elastomeric or rubber-like material that allows the line securement straps 214 to stretch over the securement knobs 212 while providing a resilient clamping force on the fluid lines 206.

The base 202 further includes ramps 222 associated with the securement knobs 212. Each ramp 222 extends along the top surface 210 and slopes downward and away from the corresponding line channel 208. The ramps 222 position the securement knobs 212 at a height such that, when the line securement straps 214 are engaged, the distal portions of the securement portions 216 and the finger grips 218 are located below the highest point of a fluid line 206 seated in the line channel 208. This geometry helps ensure that the fluid line 206 is pressed downward into the line channel 208 and that the line securement strap 214 does not become the highest point of the assembly, thereby reducing the likelihood of unintended snagging or displacement of the fluid line 206.

To assist a user in locating the line channels 208 from different viewing angles, the base 202 may include bump indicators 224 on a first side surface 226 and a second side surface 228 of the base 202. The bump indicators 224 are aligned with the line channels 208 and may be formed as raised or textured features that provide both visual and tactile cues corresponding to the positions of the line channels 208. For example, a clinician may feel along the first side surface 226 or second side surface 228 to determine the location of each line channel 208 without needing to visually inspect the top surface 210, which may be partially obscured by other equipment.

As illustrated in FIGS. 2B and 2D, each securement knob 212 includes a line engagement overhang 230 on a side of the securement knob 212 adjacent to the corresponding line securement strap 214. The line engagement overhang 230 projects outward over the associated line channel 208 so that, when the fluid line 206 is seated in the line channel 208 and the line securement strap 214 is engaged, a portion of the fluid line 206 is captured beneath the line engagement overhang 230. The securement knob 212 also includes a strap engagement overhang 232 on a side of the securement knob 212 opposite the line securement strap 214. The strap engagement overhang 232 is configured to capture and retain the finger grip 218 and distal end of the securement portion 216 when the line securement strap 214 is pulled over the securement knob 212. In use, the line securement strap 214 is stretched and pulled over the fluid line 206 and the securement knob 212 so that the securement knob 212 protrudes through the slot 220. The finger grip 218 is then positioned beneath the strap engagement overhang 232, which retains the line securement strap 214 in a secured condition and prevents it from unintentionally releasing under tension or when bumped.

The geometry of each line channel 208 may be further defined by a channel slope 234 and a line receiver 236 formed in the base 202 adjacent to a bottom region of the securement knob 212. The channel slope 234 may angle downward from a region nearer the line securement strap 214 toward the line receiver 236. The channel slope 234 and line receiver 236 may be shaped such that the contour of the channel slope 234 and line receiver 236 generally mirrors an outer contour of a smaller-diameter fluid line 206 expected to be used with the fluid line management module 200, while providing additional depth to accommodate comparatively larger-diameter fluid lines 206. As schematically indicated in FIG. 2D, the channel slope 234 and line receiver 236 cooperate with the line engagement overhang 230 and the line securement strap 214 to define an envelope that can securely hold fluid lines 206 having a range of outer diameters between a minimum outer diameter and a maximum outer diameter. When the line securement strap 214 is engaged, smaller-diameter fluid lines 206 are pressed into intimate contact with the channel slope 234 beneath the line engagement overhang 230, and larger-diameter fluid lines 206 partially occupy the line receiver 236 while still being captured beneath the line engagement overhang 230 and the line securement strap 214.

In some embodiments, the channel slope 234 may be sloped in the opposite direction toward the line securement strap 214. The pulling of the line securement strap 214 into engagement with the securement knob 212 may pull the fluid line 206 into engagement with the securement knob 212, with at least a portion of the fluid line 206 being under the line engagement overhang 230.

In some embodiments, the materials, lengths, and cross-sectional dimensions of the securement knobs 212, the line securement straps 214, and the base 202 are selected so that the line securement straps 214 can be repeatedly stretched over the securement knobs 212 without exceeding the elastic range of the strap material, maintaining consistent clamping forces over the life of the device. The wavy profile of the base 202, the arrangement of the line channels 208, and the combination of the line engagement overhangs 230, strap engagement overhangs 232, channel slopes 234, and line receivers 236 together allow the fluid line management module 200 to securely hold multiple fluid lines 206 of different sizes in a compact, organized configuration while facilitating rapid visual identification and one-handed engagement and release of individual lines.

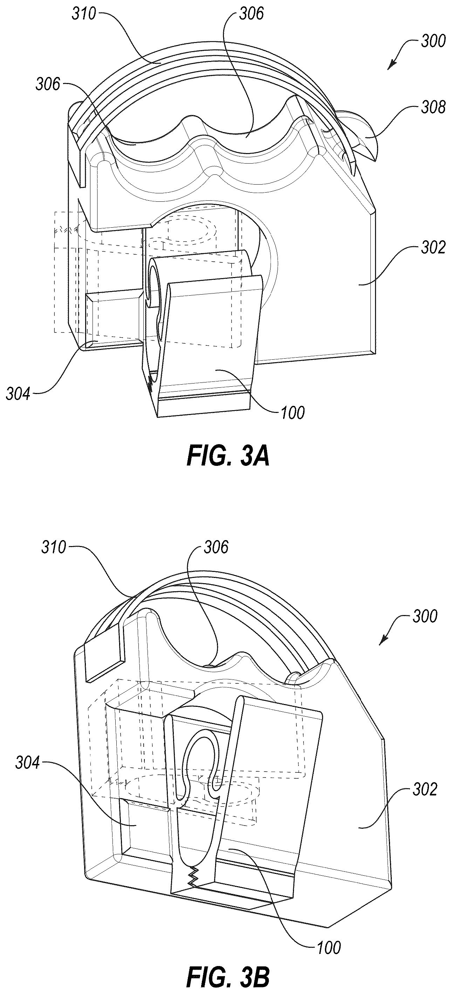

FIG. 3A is a top perspective view of a respiratory line management module 300 of a medical line management system, and FIG. 3B is a bottom perspective view of the respiratory line management module 300. The respiratory line management module 300 may be a component part of the medical line management system and may be used, for example, to organize and support respiratory lines extending between a patient and a ventilator, oscillator, or other respiratory support device.

The respiratory line management module 300 comprises a base 302 to which the attachment clip 100 may be mounted. The attachment clip 100 may be rotatably mounted to the base 302 so that the respiratory line management module 300 can be oriented in different angular positions relative to a support structure while the attachment clip 100 remains engaged with that structure. In some embodiments, a clip guide protrusion 304 is provided on a surface of the base 302. The clip guide protrusion 304 may cooperate with a mating feature on the attachment clip 100 (for example, with the foot 116 or base 112 of the attachment clip 100) to bias the attachment clip 100 into one or more preferred orientations relative to the base 302, such as discrete positions approximately 90 degrees apart, while still allowing the clinician to overcome the bias and rotate the attachment clip 100 when desired.

One or more respiratory line channels 306 may be formed on an upper surface of the base 302 of the respiratory line management module 300. As shown in FIGS. 3A-3B, the respiratory line management module 300 may comprise two respiratory line channels 306, for example to receive inspiratory and expiratory limbs of a respiratory circuit. However, any desired number of respiratory line channels 306 may be provided. The respiratory line channels 306 may be sized and shaped to accommodate respiratory lines for a given application, such as neonatal respiratory tubing, and may optionally be configured to accept lines of different outer diameters (e.g., standard ventilator tubing and oscillator tubing) while still providing secure support.

A securement knob 308 may be provided on a surface of the base 302 adjacent to the respiratory line channels 306. The securement knob 308 may be configured to interface with a line securement strap 310. In the illustrated embodiment, a single line securement strap 310 may extend across both of the respiratory line channels 306 so that, when the line securement strap 310 is engaged with the securement knob 308, the respiratory lines in both channels 306 are secured together as a unit. Securing the inspiratory and expiratory limbs together in this manner may help maintain their relative arrangement and reduce the risk of individual lines being displaced during patient movement.

The securement knob 308 and the line securement strap 310 may be generally similar in construction and function to the securement knob and line securement strap described above with respect to the fluid line management module 200, but may be sized based on the dimensions of the respiratory lines used in a given application. For example, the line securement strap 310 may include a securement portion and finger grip defining a slot configured to stretch over and receive the securement knob 308, and the securement knob 308 may include overhang regions that extend at least partially over the respiratory line channels 306 and capture the line securement strap 310 when engaged. The materials and geometry of the securement knob 308 and the line securement strap 310 may be selected so that the line securement strap 310 can be repeatedly stretched over the securement knob 308 without exceeding the elastic range of the strap material, thereby providing a consistent clamping force on the respiratory lines.

The fluid line management module 200 and the respiratory line management module 300 may thus cooperate to manage fluid lines and respiratory lines for a patient. Each of the fluid line management module 200 and the respiratory line management module 300 comprises an attachment clip 100 that facilitates attaching and detaching the respective module to and from external objects, such as bedside attachments and family holding attachments described elsewhere herein. In this manner, if a patient needs to be moved-such as in a neonatal care unit where an infant is moved from a bed to be held by a caregiver in a chair-the fluid line management module 200 and the respiratory line management module 300, along with the fluid lines and the respiratory lines held therein, may be easily moved with the patient from location to location without disturbing the securement of the lines.

To facilitate such movement, the medical line management system may comprise holding attachments that support the fluid line management module 200 and the respiratory line management module 300 at various locations. Such holding attachments may be configured to easily interface with the attachment clips 100 of the fluid line management module 200 and the respiratory line management module 300.

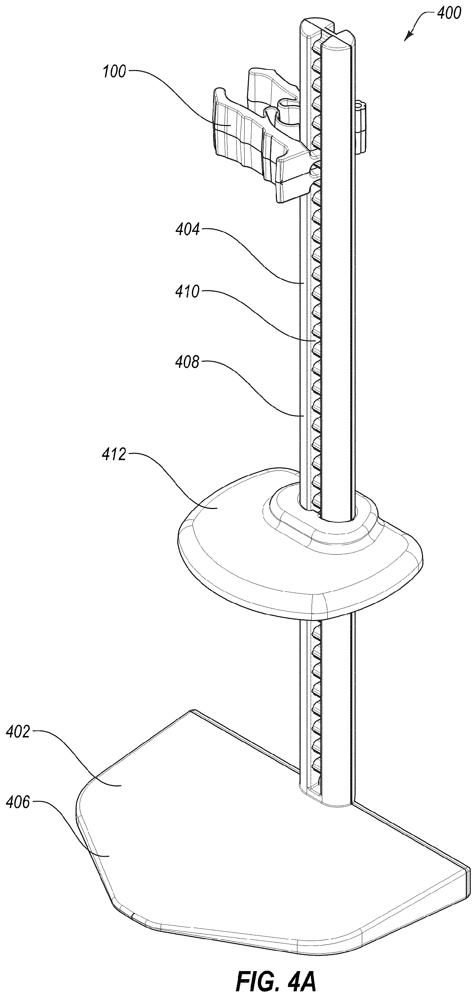

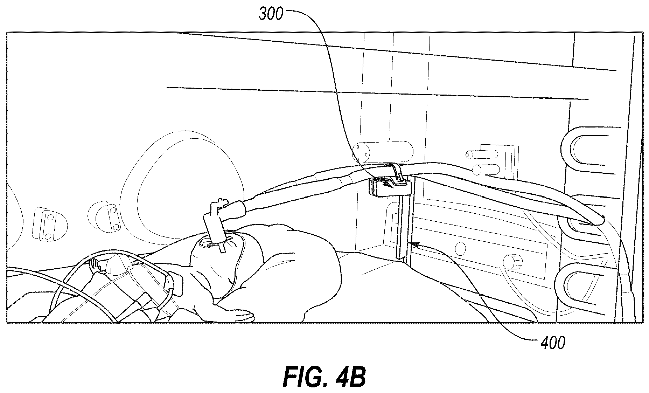

FIG. 4A is a perspective view of a bedside attachment 400 of a medical line management system, and FIG. 4B shows the bedside attachment 400 in an exemplary environment of use. The bedside attachment 400 may comprise a stable base 402 and a tower 404 extending upward from the stable base 402. The stable base 402 is a relatively flat, plate-like member that is, for example, configured to slide underneath a mattress of a neonatal bed or incubator. To facilitate this, the stable base 402 may comprise an inclined surface 406 at an outside edge of the stable base 402 opposite the tower 404, allowing that edge to be easily slid underneath the mattress with minimal lifting of the mattress.

The tower 404 may be formed to have a height that extends above the top surface of the mattress when the stable base 402 is positioned beneath the mattress. The tower 404 is configured to receive one or more attachment clips 100 of one or more line management modules (e.g., the line management modules 200, 300) so that the line management modules are supported adjacent the bedside. The tower 404 may thus be an example of a clip receiving structure. To this end, the tower 404 may comprise a tower channel 408 that extends at least partially along the height of the tower 404. The tower channel 408 may define a securement column profile that corresponds to a gripping or foot portion of the attachment clip 100 so that the attachment clip 100 can be removably engaged with the tower 404 in a stable, guided manner. For example, the tower channel 408 may include channel pockets 410 that are sized and shaped receive corresponding engagement surfaces (e.g., interlocking teeth 114) of the attachment clip 100, thereby resisting lateral twisting and inadvertent disengagement when the line management module is loaded with lines. The combination of the tower channel 408 and the channel pockets 410 may define a columnar profile that securely captures the attachment clip 100 at different vertical positions along the height of the tower 404.

The bedside attachment 400 may further comprise a top plate 412 associated with the tower 404. The top plate 412 may extend laterally from the tower 404 and may be movable along at least a portion of the height of the tower 404 between an elevated position and a lowered position. In use, when the stable base 402 is positioned beneath a mattress, the top plate 412 may be slid downward along the tower 404 until the top plate 412 rests on or adjacent to an upper surface of the mattress. In this configuration, the mattress may be sandwiched between the stable base 402 and the top plate 412, thereby providing additional stability against tipping or rocking of the tower 404 when the line management modules and associated lines are attached or adjusted. In some embodiments, the top plate 412 may be formed from a translucent material to allow a user to visually confirm the position of the top plate 412 relative to the mattress and surrounding equipment.

As shown schematically in FIG. 4B, the bedside attachment 400 may be used to support, for example, a respiratory line management module 300 at the side of a neonatal bed. The attachment clip 100 of the respiratory line management module 300 may be engaged with the tower channel 408 of the tower 404 such that the respiratory lines secured to the respiratory line management module 300 are supported within a desired proximity of the patient while the stable base 402 and top plate 412 cooperate to stabilize the bedside attachment 400 relative to the mattress. In this manner, the bedside attachment 400 provides a stable mounting location for the line management modules at the bedside, while still allowing the line management modules to be easily detached and reattached as the patient is moved between the bed and other locations (such as a family holding attachment described elsewhere herein).

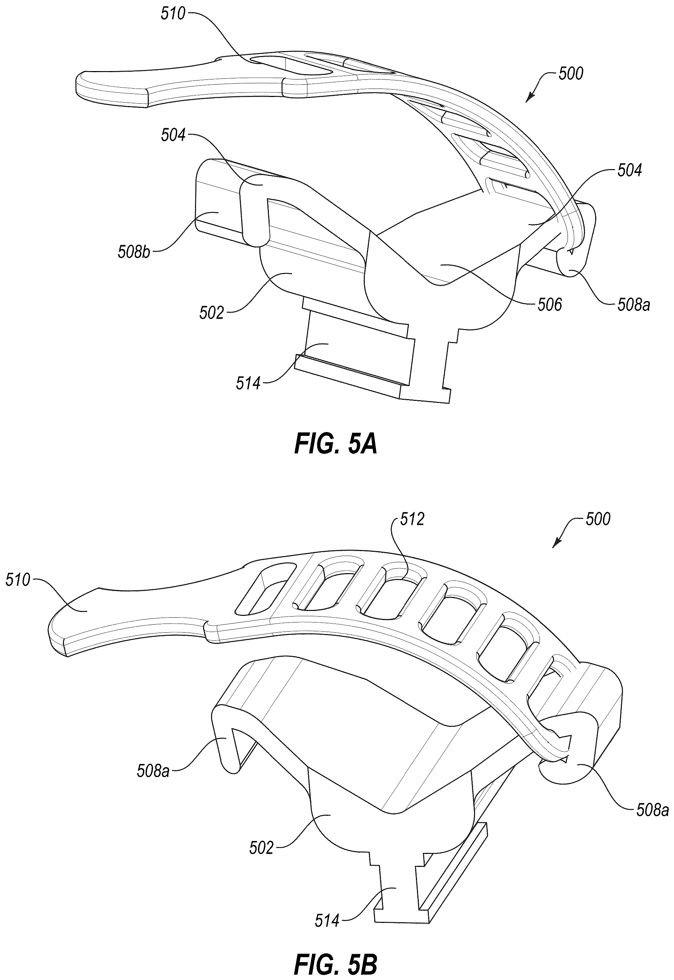

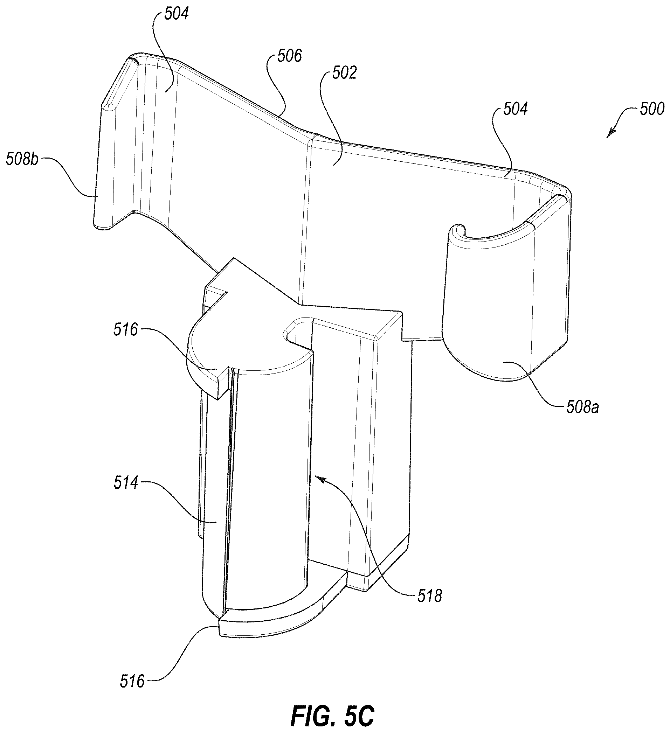

FIG. 5A is a side perspective view of a family holding attachment 500 of a medical line management system, FIG. 5B is a top perspective view of the family holding attachment 500, and FIG. 5C is a top perspective view of the family holding attachment 500 with an alternative clip securement column 514. Referring to FIGS. 5A-5C, the family holding attachment 500 is configured to support one or more line management modules (for example, the fluid line management module 200 and/or the respiratory line management module 300) when a patient is held away from the bed or incubator, such as in a caregiver's arms. The family holding attachment 500 may be configured to be removably secured to an external object, such as an IV pole or a back of a chair.

The family holding attachment 500 includes a base 502 and at least one arm 504 extending from the base 502. In the embodiment of FIGS. 5A and 5B, the arms 504 extend generally away from the base 502 and cooperate with the base 502 to define a receiving channel 506. The receiving channel 506 is configured to receive a structural member of an external object, such as a chair arm, bed rail, IV pole, a wrist or arm of a caregiver, or another support surface. The cross-sectional shape and dimensions of the receiving channel 506 may be selected based on the geometry of the intended support structure so that, when the family holding attachment 500 is mounted, the base 502 and arms 504 cradle and stabilize the family holding attachment 500 on the support.

A clip securement column 514 is formed on or adjacent to the base 502. The clip securement column 514 defines a profile that is configured to be engaged by the attachment clip 100. The clip securement column 514 may provide a standardized rail or column along which the attachment clip 100 can be seated, allowing a line management module attached via the attachment clip 100 to be mounted to the family holding attachment 500 in a stable and repeatable orientation. The clip securement column 514 is another example of a clip receiving structure configured to receive the attachment clip 100 to facilitate attachment of a line management module.

The family holding attachment 500 further includes at least one attachment strap 510. The attachment strap 510 may be fixed at or near one side of the receiving channel 506 and configured to extend around the external object received in the receiving channel 506. For example, the attachment strap 510 may be anchored proximate one of the arms 504 and may be flexible so that a distal portion of the attachment strap 510 can be wrapped around the external object (e.g., IV pole or chair back) and brought back toward the base 502. The attachment strap 510 includes a plurality of slots 512 along its length. The slots 512 are spaced so that different ones of the slots 512 may be selectively engaged depending on the size of the external object, thereby allowing the effective loop length of the attachment strap 510 around the external object to be adjusted. In some embodiments, the overall length of the attachment strap 510 and the spacing of the slots 512 are selected based on the expected elongation characteristics of the strap material, so that the strap can be securely tightened around a range of support sizes while remaining within an elastic operating range.

To facilitate use of the attachment strap 510, the family holding attachment 500 may include a first hook 508a and a second hook 508b positioned on or near the arms 504. In one example, the first hook 508a is configured to attach to the attachment strap 510. When the family holding attachment 500 is to be secured to an external object, the attachment strap 510 may be routed around the external object and then engaged with the second hook 508b, for example by inserting a selected one of the slots 512 over the second hook 508b. The distal end of the attachment strap 510 may include a pull tab portion that mimics the shape and texturing of pull tabs on other straps used in the system, such as straps of the fluid line management module 200 or the respiratory line management module 300, to provide a consistent user interface.

FIG. 5C illustrates an embodiment of the family holding attachment 500. In the embodiment, the clip securement column 514 may include one or more edge stoppers 516 near its upper and/or lower ends. The edge stoppers 516 form enlarged or raised regions at the ends of the clip securement column 514 that act as physical stops to reduce the likelihood that the attachment clip 100 will slide off the end of the clip securement column 514 when the family holding attachment 500 is loaded or bumped. Between the edge stoppers 516, the clip securement column 514 may include a column groove 518 that extends along the length of the column. The column groove 518 is sized and shaped to receive a complementary portion of the attachment clip 100 (for example, the interlocking teeth 114 of the attachment clip 100). The combination of the column groove 518 and the edge stoppers 516 thereby defines a clip securement column 514 that guides and retains the attachment clip 100 along its length.

In use, when a patient is moved from a first location, such as a bed or incubator where the line management modules 200, 300 are engaged with a bedside attachment, to a second location, such as a chair for family holding, the attachment clip 100 of each line management module can be released from the bedside attachment (e.g., the bedside attachment 400) and engaged with the clip securement column 514 of the family holding attachment 500. The receiving channel 506 and attachment strap 510 cooperate to secure the family holding attachment 500 to the external support (e.g., chair arm or IV pole), and the clip securement column 514, together with the edge stoppers 516 and column groove 518 in the embodiment of FIG. 5C, cooperate to retain the attachment clips 100 on the family holding attachment 500. The attachment clip 100 may also be engaged with other objects such as fabric (e.g., clothing of a caregiver, sheets, blankets, etc.). When the patient is returned to the original location, the attachment clips 100 can be released from the family holding attachment 500 and re-engaged with the bedside attachment without disturbing the arrangement of the fluid and respiratory lines held in the line management modules, thereby keeping such lines organized and safe during movement of the patient.

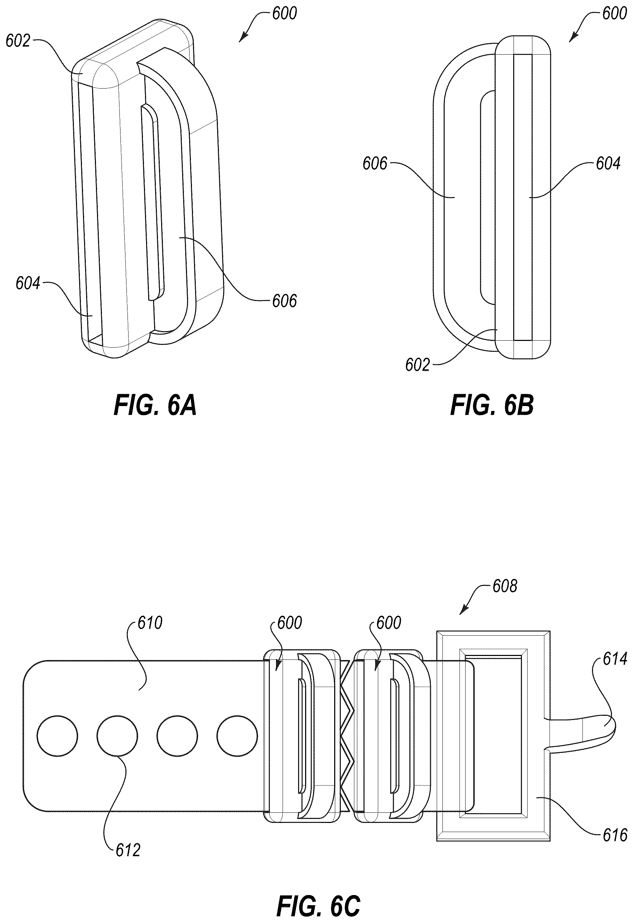

The medical line management system may include other accessories to facilitate movement of the fluid and respiratory lines while keeping such lines safe and organized. FIG. 6A is a perspective view of a sliding securement tab 600, and FIG. 6B is a side view of the sliding securement tab 600. FIG. 6C is a perspective view of a family holding attachment 608.

Referring to FIGS. 6A and 6B, the sliding securement tab 600 comprises a body 602 and a slot 604 formed through the body 602. The slot 604 is sized to allow the sliding securement tab 600 to be threaded onto an elongated flexible member, such as a belt 610 of the family holding attachment 608 or another strap-like device. The sliding securement tab 600 may be formed from a relatively rigid material, such as a molded polymer, so that it maintains its shape and provides a stable mounting surface when loaded by an attached line management module.

A clip securement column 606 (a clip receiving structure) extends from the body 602. The clip securement column 606 defines a profile configured to be removably engaged by an attachment clip 100 of one of the line management modules described herein. In some embodiments, the cross-sectional shape of the clip securement column 606 is substantially similar to the clip securement column 514 of the family holding attachment 500 or a portion of the profile of the tower channel 408 of the bedside attachment 400 described above, so that the same attachment clip 100 can engage any of these structures in a consistent manner. When an attachment clip 100 is engaged with the clip securement column 606, the corresponding fluid line management module 200 or respiratory line management module 300 is thereby supported by the sliding securement tab 600. Because the slot 604 allows the sliding securement tab 600 to slide along the underlying belt or strap, the position of the attached module can be adjusted along the length of that belt or strap as needed.

Referring to FIG. 6C, the family holding attachment 608 comprises a belt 610 having a length sufficient to surround an object on which it is to be mounted, such as a chair back, mattress segment, rail, or a portion of a caregiver's body. The belt 610 may be formed from a flexible, resilient material that can conform to the shape of the object and provide frictional engagement when tensioned. The belt 610 includes a plurality of holes 612 along at least a portion of its length. The holes 612 are positioned and spaced such that the belt 610 can be tightened around objects of different sizes while remaining within a desired elastic operating range of the belt material.

A buckle 616 is provided at one end of the belt 610, and a hook 614 is associated with the buckle 616. In use, the belt 610 is wrapped around the object, and a selected one of the holes 612 is placed over the hook 614 of the buckle 616 to secure the belt 610 in a closed loop configuration. By selecting different ones of the holes 612, a clinician can adjust the tension applied by the belt 610 to securely hold the family holding attachment 608 in place on the underlying object.

One or more sliding securement tabs 600 may be threaded onto the belt 610 by passing the belt 610 through the slot 604 of each sliding securement tab 600. Because the slot 604 allows the sliding securement tabs 600 to translate along the belt 610, the clinician can position the sliding securement tabs 600 at desired locations along the circumference of the belt 610. The clip securement columns 606 of the sliding securement tabs 600 thereby provide one or more standardized mounting points along the belt 610 to which attachment clips 100 of the line management modules can be removably attached. In this manner, the family holding attachment 608 and sliding securement tabs 600 cooperate to create a flexible, adjustable support that can be mounted to a variety of objects and that can carry one or more line management modules at user-selected positions while the patient is moved or repositioned, without disturbing the securement of the fluid and respiratory lines held in those modules.

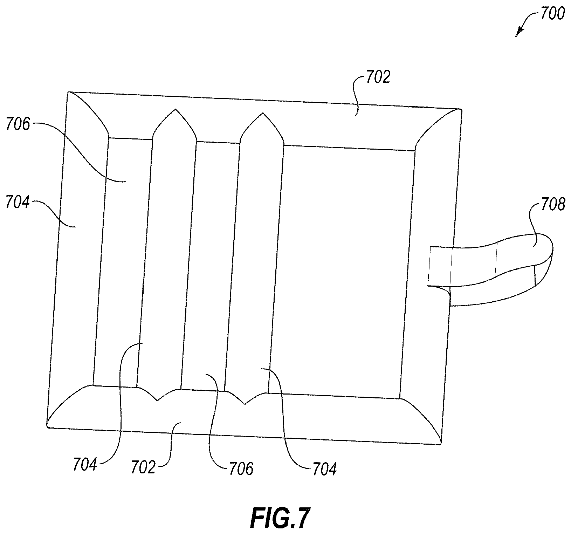

In some embodiments, the buckle 616 of the family holding attachment 608 may be replaced with an alternative buckle 700 shown in FIG. 7. The buckle 700 may comprise lateral extension members 702 and a plurality of cross members 704 extending between the lateral extension members 702 to form a frame. The cross members 704 define a plurality of slots 706, each configured to receive and interface with the belt 610. The belt 610 may be woven between the cross members 704 and through the slots 706 in a selected pattern so that friction between the belt 610 and the buckle 700 provides reversible securement of the belt 610 to the buckle 700. This arrangement allows the belt 610 to be tightened around an underlying object and held in place without permanent fastening. When the belt 610 needs to be cleaned or replaced, the belt 610 may be unwoven from the slots 706 and separated from the buckle 700 without tools.

The buckle 700 further comprises a hook 708 that interfaces with one of the plurality of holes 612 of the belt 610. After the belt 610 is woven through the slots 706 to establish a desired length and routing, a selected one of the holes 612 may be placed over the hook 708 to complete the securement of the family holding attachment 608 around the underlying object.

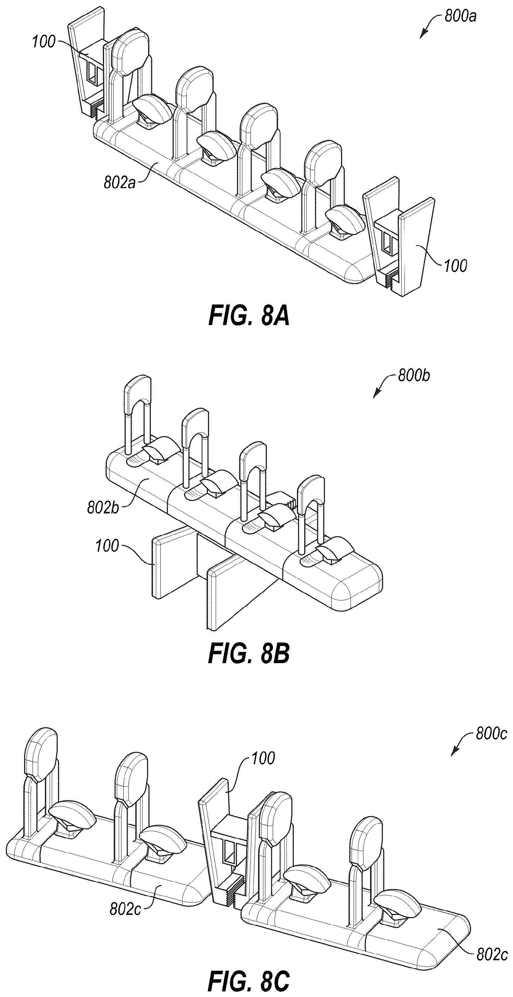

The medical line management system and its components described herein are not limited to the embodiments shown above. Several modifications may be within the scope of the disclosure, including alternative arrangements of the line management modules and attachment clips. FIGS. 8A-8D illustrate example alternative fluid line management modules.

FIG. 8A is a perspective view of a fluid line management module 800a. The fluid line management module 800a may comprise a base 802a that has a substantially rectangular shape as opposed to the wavy shape of the base 202 of the fluid line management module 200 shown in FIGS. 2A-2D. The fluid line management module 800a may further comprise two attachment clips 100, with one attachment clip 100 disposed at each end of the base 802a. In some embodiments, the line-handling features of the fluid line management module 800a (such as line channels, securement knobs, and securement straps) may be generally similar to those of the fluid line management module 200, but the presence of two attachment clips 100 may provide increased stability or allow the module 800a to be supported on two spaced-apart mounting locations if desired.

FIG. 8B is a perspective view of a fluid line management module 800b. The fluid line management module 800b may comprise a base 802b with a generally rectangular shape. An attachment clip 100 of the fluid line management module 800b may be attached to a bottom surface of the base 802b. Positioning the attachment clip 100 on the bottom surface can allow the top surface of the base 802b-where line channels, securement knobs, and securement straps may be located-to remain unobstructed and easily accessible while the module 800b is mounted close to a support structure.

FIG. 8C is a perspective view of a fluid line management module 800c. The fluid line management module 800c may comprise a pair of bases 802c that are spaced apart from one another and that each attach to an attachment clip 100 disposed between them. In one example, the attachment clip 100 may be centrally located between the bases 802c so that the fluid line management module 800c can be supported at a single attachment point while providing line-handling features on both sides of the attachment clip 100.

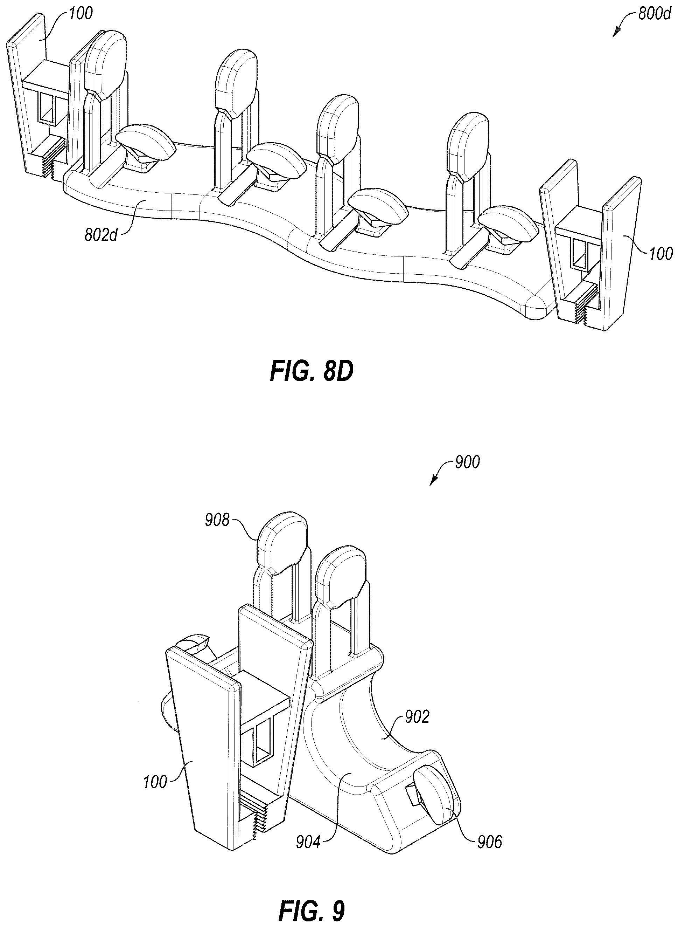

FIG. 8D is a perspective view of a fluid line management module 800d. The fluid line management module 800d may comprise a base 802d and a pair of attachment clips 100 connected at ends of the base 802d, respectively. The fluid line management module 800d may, in some embodiments, include line-handling features similar to those of the fluid line management module 200 and may illustrate that the number, placement, and orientation of attachment clips 100 on a given base can be varied to suit different mounting and stability requirements while still using the same standardized attachment clip interface. The embodiments shown in FIGS. 8A-8D thereby demonstrate that the geometry of the bases and the arrangement of attachment clips 100 can be tailored for particular use cases, and that different modifications may be within the scope of the disclosure.

Similarly, a respiratory line management module is not limited to the respiratory line management module 300 shown above with reference to FIGS. 3A and 3B. FIG. 9 illustrates an alternative respiratory line management module 900. The respiratory line management module 900 comprises a base 902 and an attachment clip 100. The attachment clip 100 may correspond to the attachment clip 100 described above and may be disposed on a front surface of the base 902 to allow the respiratory line management module 900 to be removably mounted to the various supports of the medical line management system.

Two channels 904 are formed on a top surface of the base 902, for example with one channel 904 disposed on each lateral side of the top surface. Each channel 904 is configured to receive at least a portion of a respiratory line. A securement knob 906 and a securement strap 908 are provided for each channel 904. The securement knobs 906 and securement straps 908 may be generally similar in structure and function to the securement knobs and securement straps described above in connection with other modules, and may be configured so that, when a securement strap 908 is stretched over its corresponding securement knob 906, a respiratory line received in the channel 904 is retained in a secured position. The embodiment of FIG. 9 thereby illustrates that the number and placement of respiratory line channels, as well as the location of the attachment clip 100 on the base, can be varied while still providing secure support and organization of respiratory lines.

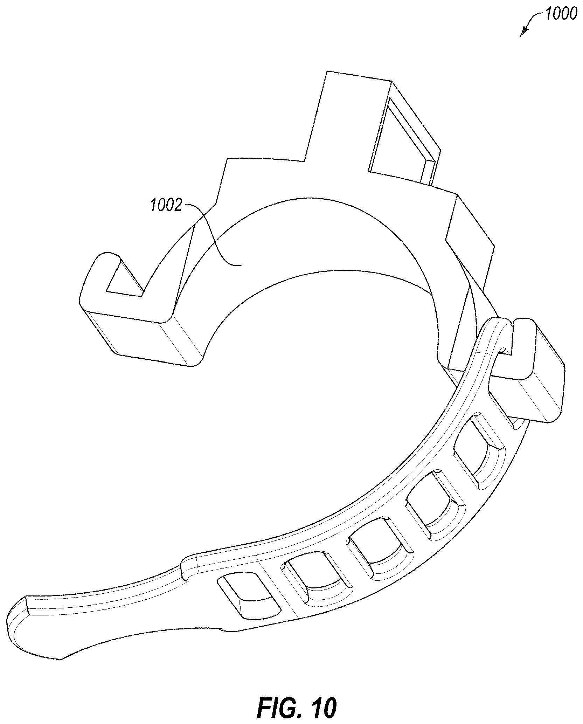

FIG. 10 shows a family holding attachment 1000. The family holding attachment 1000 may be structurally and functionally similar to the family holding attachment 500 described above, except as noted below. In particular, the family holding attachment 1000 includes a receiving channel 1002 having a rounded cross-sectional profile. The receiving channel 1002 is configured to receive a generally cylindrical support member, such as an IV pole or similar post, and to be secured to the support when an attachment strap is tightened around the support.

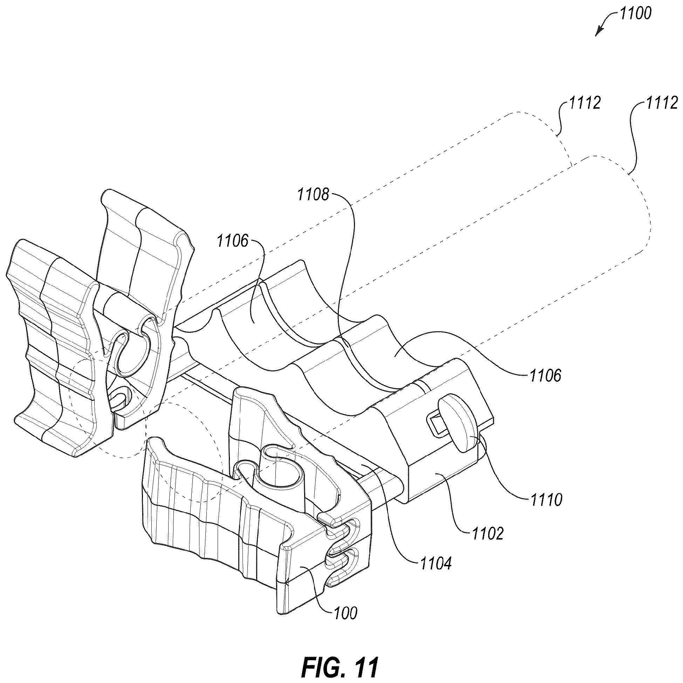

FIG. 11 illustrates a respiratory line management module 1100. The respiratory line management module 1100 may be a component part of the medical line management system and may be used, for example, to support respiratory lines extending between a patient and a ventilator, oscillator, or other respiratory support device near the patient interface. The respiratory line management module 1100 comprises a base 1102 to which the attachment clip 100 may be mounted.

The base 1102 includes a slot 1104 that extends along at least a portion of the length of the base 1102. The slot 1104 is configured to receive a portion of the attachment clip 100 so that the attachment clip 100 can translate relative to the base 1102 between different positions along the slot 1104. In some embodiments, the attachment clip 100 may also be rotatably mounted to the base 1102, such that the respiratory line management module 1100 can be both rotated and translated relative to an underlying support structure (for example, a bedside attachment or family holding attachment) without disconnecting the attachment clip 100 from that support. This combination of rotation and translation enables a clinician to adjust the position of the respiratory line management module 1100 to accommodate different patient positions and equipment layouts while maintaining the securement of the respiratory lines.

The base 1102 further defines one or more respiratory line channels 1106 configured to receive a portion of one or more respiratory lines 1112. In the illustrated embodiment, the respiratory line channel 1106 extends generally along the top of the base 1102 and is sized to cradle an inspiratory limb and/or an expiratory limb of a respiratory circuit. The respiratory line channel 1106 includes engagement surfaces 1108 that at least partially conform to an outer contour of the respiratory lines 1112. The engagement surfaces 1108 may be arranged to contact the respiratory lines 1112 over multiple angular positions around their circumference so as to distribute support forces and help maintain patency of the respiratory lines 1112 while resisting unintended movement or sagging. The engagement surfaces 1108 may be continuous or segmented surfaces, such as one or more ribs, formed in or on the base 1102.

A securement knob 1110 is disposed on the base 1102 adjacent the respiratory line channel 1106. The securement knob 1110 may be configured to interface with a line securement strap, such as the line securement strap 310 described above in connection with the respiratory line management module 300. In use, the respiratory lines 1112 are positioned in the respiratory line channel 1106 against the engagement surfaces 1108, and the line securement strap is stretched over the respiratory lines 1112 and engaged with the securement knob 1110 so that a portion of each respiratory line 1112 is held between the strap and the engagement surfaces 1108. In this secured condition, the respiratory line management module 1100 supports and organizes the respiratory lines 1112 while the attachment clip 100 and slot 1104 allow the overall position and orientation of the module 1100 to be adjusted relative to the patient and surrounding equipment without disturbing the securement of the respiratory lines 1112.

The embodiments of the disclosure described above and illustrated in the accompanying drawings do not limit the scope of the disclosure, which is encompassed by the scope of the appended claims and their legal equivalents. Any equivalent embodiments are within the scope of this disclosure. Indeed, various modifications of the disclosure, in addition to those shown and described herein, such as alternate useful combinations of the elements described, will become apparent to those skilled in the art from the description. Such modifications and embodiments also fall within the scope of the accompanying claims and equivalents.

## Claims

1. A medical line management system comprising:
a line management module configured to secure one or more medical lines; and
a family holding attachment, the line management module being removably attachable to the family holding attachment.

2. The medical line management system of claim 1, wherein the line management module comprises a base and an attachment clip connected to the base, the attachment clip configured to removably attach to a bedside attachment and the family holding attachment.

3. The medical line management system of claim 1 or claim 2, wherein the line management module comprises line channels configured to receive the one or more medical lines.

4. The medical line management system of claim 3 when dependent upon claim 2, wherein the line channels are defined by a line securement knob extending from the base and a line securement strap extending from the base and configured to removably attach to the line securement knob to secure the one or more medical lines in the line channels.

5. The medical line management system of any one of the preceding claims, further comprising a bedside attachment, the line management module being removably attachable to the bedside attachment; wherein the bedside attachment comprises a stable base and a tower configured to receive the line management module, wherein the stable base optionally comprises a top plate configured to rest adjacent to a top surface of the mattress.

6. The medical line management system of any one of the preceding claims, wherein the family holding attachment comprises an attachment strap configured to mount the family holding attachment to an external object, wherein the family holding attachment optionally comprises a first arm and a second arm, the attachment strap being attached to the first arm and removably attachable to the second arm.

7. The medical line management system of any one of the preceding claims, wherein the family holding attachment comprises a clip receiving structure configured to receive the line management module, wherein the family holding attachment optionally comprises a sliding securement tab, the sliding securement tab comprising the clip receiving structure to receive the line management module.

8. A method of managing and moving medical lines, the method comprising:
securing medical lines to a line management module;
moving the line management module from a bedside with the medical lines secured to the line management module; and
attaching the line management module to a family holding attachment with the medical lines secured to the line management module.

9. The method of claim 8, wherein securing the medical lines to the line management module comprises pulling a line securement strap over a securement knob to secure the medical lines between the line securement strap and the securement knob.

10. The method of claim 8 or claim 9, further comprising attaching the line management module to a bedside attachment by attaching an attachment clip of the line management module to a clip receiving structure of the bedside attachment, and wherein attaching the line management module to a family holding attachment comprises attaching the attachment clip of the line management module to a clip receiving structure of the family holding attachment.

11. A medical line management module comprising:
a base;
a plurality of line channels disposed on the base, each line channel configured to receive a medical line therein;
at least one line securement strap configured to secure the medical line in each line channel; and
an attachment clip connected to the base, the attachment clip configured to mount the medical line management module to an external object.

12. The medical line management module of claim 11, wherein the attachment clip is rotatably mounted to the base to facilitate relative rotation between the base and the attachment clip, and wherein the attachment clip comprises:
interlocking teeth configured to attach to a fabric material; and
stoppers configured to limit an opening distance of the interlocking teeth.

13. The medical line management module of claim 11 or claim 12, further comprising a securement knob comprising a strap engagement overhang, the line securement strap configured to engage with the strap engagement overhang to secure the medical line in each line channel, wherein the securement knob optionally comprises a line engagement overhang that extends at least partially over a corresponding line channel.

14. The medical line management module of any one of claims 11 to 13, wherein the line securement strap comprises a securement portion that comprises two members that are connected at a distal end thereof by a finger grip.

15. The medical line management module of any one of claims 11 to 14, wherein the base comprises bump indicators disposed on a side surface thereof, the bump indicators being aligned with the line channels and/or wherein the base exhibits a wavy shape such that neighboring line channels are laterally offset from one another.
